# EUROPEAN PATENT APPLICATION

(11) **EP 4 792 725 A2**
(43) Date of publication of application: **19.08.2026**
(21) Application number: 25225356.2
(22) Date of filing: 19.12.2025
(51) Int. Cl.: A61B 1/00, A61B 1/005

(54) **ENDOSCOPE SYSTEM, CONTROL METHOD, AND PROGRAM**

(30) Priority: 14.02.2025 JP 2025022293
(71) Applicant: Evident Inspection Technologies Japan Corporation, Kamiina-gun, Nagano 399-0495 (JP)
(72) Inventor: NUMATA, Kenji, Nagano, 399-0495 (JP)
(74) Representative: K&L Gates LLP

(57) **Abstract**

An endoscope system (1) includes an insertion unit (3) including a sensor (32) at a distal end (34) and being bendable and a control unit (50). The control unit receives a mode signal indicating a mode in which the insertion unit is automatically bent. The control unit acquires data output from the sensor. The control unit determines a gravitational direction (Dg) at the distal end based on the data. The control unit outputs a control signal for bending the insertion unit such that an angle (An) between a forward direction (Dif) of the distal end and the gravitational direction (Dg) increases after the mode signal has been received.

## Description

### TECHNICAL FIELD

The present invention relates to an endoscope system, a control method, and a program.

### BACKGROUND ART

An industrial endoscope device has been used for inspection for abnormality, corrosion, and the like (endoscopic inspection) in boilers, pipes, aircraft engines, heat exchangers, and the like. A user inspects an inspection target using an endoscope device. The endoscope device includes an insertion unit for observing the inside of a subject. A user inserts the insertion unit into an inspection target and checks whether abnormalities such as cracks have occurred in the inspection target.

Sediment, oil, or the like on the bottom surface of a pipe may become attached to an observation optical system at the distal end of an insertion unit and hinder observation of a subject in inspection of the pipe. An endoscope device disclosed in Patent Document 1 has a function of sucking in liquid contamination attached to an observation optical system using the capillary phenomenon. Accordingly, a user can observe a clear image.

### Citation List

### Patent Document

Patent Document 1: Japanese Unexamined Patent Application, First Publication No. 2017-37138

### SUMMARY OF INVENTION

### Technical Problem

In the related art, when a large amount of contamination is attached to the observation optical system, it is difficult to completely remove the contamination. Since attachment of contamination to the observation optical system cannot be prevented, a user needs to perform complicated operations to move an insertion unit in a subject.

An objective of the present invention is to provide an endoscope system, a control method, and a program that can reduce foreign matter coming into contact with an observation optical system without requiring a user's complicated operations. Solution to Problem

The present invention is an endoscope system including an insertion unit that includes a sensor at a distal end and that is bendable and a control unit configured to: receive a mode signal indicating a mode in which the insertion unit is automatically bent; acquire data output from the sensor; determine a gravitational direction at the distal end based on the data; and output a control signal for bending the insertion unit such that an angle between a forward direction of the distal end and the gravitational direction increases after the mode signal has been received.

In the endoscope system according to the present invention, the control unit is configured to output a state control signal for controlling a bending state of the insertion unit such that the angle is fixed after the control signal has been output.

In the endoscope system according to the present invention, the control unit is configured to output the control signal for bending the insertion unit in a first direction and a second direction perpendicular to the first direction such that the angle increases according to an amount of twist of the insertion unit.

In the endoscope system according to the present invention, the control unit is configured to generate the control signal based on a predetermined value stored in a memory, and the predetermined value indicates a target value of the angle or a target value of an amount of increase of the angle.

In the endoscope system according to the present invention, the memory is configured to store two or more values as the predetermined value, and the control unit is configured to acquire any one of the two or more values from the memory.

In the endoscope system according to the present invention, the control unit is configured to acquire the predetermined value corresponding to a type of an optical adapter attached to the distal end from the memory.

In the endoscope system according to the present invention, the predetermined value is changeable.

In the endoscope system according to the present invention, the sensor is an acceleration sensor, and the data indicates acceleration of the distal end.

The endoscope system according to the present invention further includes an image sensor configured to generate an image, and the control unit is configured to: determine a posture of the distal end based on the gravitational direction; superimpose information indicating the posture on the image; and output the image on which the information has been superimposed to a display.

In the endoscope system according to the present invention, the sensor is an image sensor configured to generate an image as the data.

In the endoscope system according to the present invention, the control unit is configured to determine the gravitational direction based on a brightness at two or more positions in the image.

In the endoscope system according to the present invention, the control unit is configured to: determine a distance between the distal end and a subject into which the insertion unit is inserted using the image; and output the control signal for bending the insertion unit such that the distance increases.

In the endoscope system according to the present invention, the control unit is configured to: output an image for displaying a graphical user interface associated with setting of the mode to a display; and receive the mode signal when it is instructed to set the mode via the graphical user interface.

The endoscope system according to the present invention further includes an image sensor configured to generate an image, and the control unit is configured to: calculate a component in the gravitational direction on an imaging surface of the image sensor; calculate an angle between a direction of the component on the imaging surface and a reference direction on the imaging surface; and correct the image by rotating the image by the angle.

The present invention is a control method including: receiving a mode signal indicating a mode in which an insertion unit including a sensor at a distal end and being bendable is automatically bent; acquiring data output from the sensor; determining a gravitational direction at the distal end based on the data; and outputting a control signal for bending the insertion unit such that an angle between a forward direction of the distal end and the gravitational direction increases after the mode signal has been received.

The present invention is a program causing a computer to execute: a step of receiving a mode signal indicating a mode in which an insertion unit including a sensor at a distal end and being bendable is automatically bent; a step of acquiring data output from the sensor; a step of determining a gravitational direction at the distal end based on the data; and a step of outputting a control signal for bending the insertion unit such that an angle between a forward direction of the distal end and the gravitational direction increases after the mode signal has been received.

### Advantageous Effects of Invention

According to the present invention, the endoscope system, the control method, and the program can reduce foreign matter coming into contact with an observation optical system without requiring a user's complicated operations.

### BRIEF DESCRIPTION OF DRAWINGS

[FIG. 1] A block diagram showing an example of the configuration of an endoscope system according to a first embodiment of the present invention.
[FIG. 2] A diagram showing an example of a state of an insertion unit in the first embodiment of the present invention.
[FIG. 3] A diagram showing an example of an image displayed on a display in the first embodiment of the present invention.
[FIG. 4] A flowchart showing an example of a procedure of a bending control process in the first embodiment of the present invention.
[FIG. 5] A flowchart showing an example of a procedure of an impact detection process in the first embodiment of the present invention.
[FIG. 6] A flowchart showing an example of a procedure of a state update process in the first embodiment of the present invention.
[FIG. 7] A flowchart showing an example of a procedure of a bending locking process in the first embodiment of the present invention.
[FIG. 8] A diagram showing an example of a state of the insertion unit in the first embodiment of the present invention.
[FIG. 9] A diagram showing an example of an image displayed on the display in the first embodiment of the present invention.
[FIG. 10] A diagram showing an example of a state of the insertion unit in the first embodiment of the present invention.
[FIG. 11] A diagram showing an example of an image displayed on the display in the first embodiment of the present invention.
[FIG. 12] A diagram showing an example of a state of the insertion unit in the first embodiment of the present invention.
[FIG. 13] A diagram showing an example of an image displayed on the display in the first embodiment of the present invention.
[FIG. 14] A diagram showing an example of a state of the insertion unit in the first embodiment of the present invention.
[FIG. 15] A diagram showing an example of an image displayed on the display in the first embodiment of the present invention.
[FIG. 16] A diagram showing a relationship between the amount of twist of the insertion unit and the amount of pulling of a wire for bending the insertion unit in the first embodiment of the present invention.
[FIG. 17] A diagram showing an example of a state of the insertion unit in the first embodiment of the present invention.
[FIG. 18] A diagram showing an example of an image displayed on the display in the first embodiment of the present invention.
[FIG. 19] A diagram showing an example of a relationship between an imaging surface of an imaging device and a gravitational direction in the first embodiment of the present invention.
[FIG. 20] A block diagram showing an example of the configuration of an endoscope system according to a first modified example of the first embodiment of the present invention.
[FIG. 21] A block diagram showing an example of the configuration of an endoscope system according to a second modified example of the first embodiment of the present invention.
[FIG. 22] A block diagram showing an example of the configuration of an endoscope system according to a second embodiment of the present invention.
[FIG. 23] A flowchart showing an example of a procedure of a bending control process in the second embodiment of the present invention.
[FIG. 24] A diagram showing an example of an image generated by an imaging device in the second embodiment of the present invention.

### DESCRIPTION OF EMBODIMENTS

Hereinafter, embodiments of the present invention will be described with reference to the drawings. A subject in the embodiments of the present invention is an industrial product. In the following examples, the subject is a pipe.

### (First embodiment)

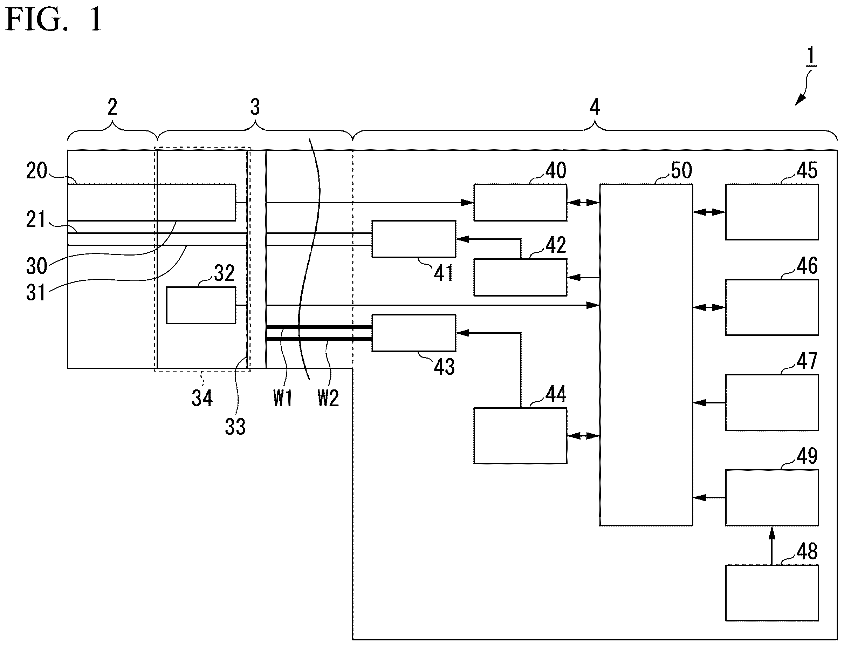

A first embodiment of the present invention will be described. FIG. 1 shows the configuration of an endoscope system 1 according to the first embodiment. The endoscope system 1 shown in FIG. 1 includes an optical adapter 2, an insertion unit 3, and a main body 4.

The optical adapter 2 is attached to a hard distal end portion 34 including the distal end of the insertion unit 3. The optical adapter 2 includes an imaging lens unit 20 and an illumination lens unit 21. The imaging lens unit 20 includes one or more imaging lenses and receives light reflected by an inner surface of a pipe. The illumination lens unit 21 includes one or more illumination lenses and irradiates the inner surface of the pipe with illumination light.

The optical adapter 2 is interchangeable, and two or more optical adapters different in an observation direction, an angle of view, a focal position, or the like can be used. For example, a direct-view optical adapter for observing a subject in a direction parallel to a longitudinal direction of the insertion unit 3 may be used. Alternatively, a side-view optical adapter for observing a subject in a direction perpendicular to the longitudinal direction of the insertion unit 3 may be used.

The insertion unit 3 is inserted into a pipe that is an observation target. The insertion unit 3 has a thin and long soft tube shape and is bendable. For example, the diameter of the insertion unit 3 ranges from 4 mm to 6 mm. The insertion unit 3 includes an imaging device 30, a light guide 31, a sensor 32, and a bending portion 33. The imaging device 30, the light guide 31, and the sensor 32 are disposed in the distal end portion 34.

The imaging device 30 is an image sensor such as a charge-coupled device (CCD) image sensor or a complementary metal-oxide-semiconductor (CMOS) image sensor. Light incident on the imaging lens unit 20 of the optical adapter 2 passes through the imaging lens unit 20 and forms an optical image on an imaging surface of the imaging device 30. Two or more pixels are arranged on the imaging surface. The imaging device 30 generates an image (a LAW image) based on an optical image formed on the imaging surface. Each pixel includes a photoelectric conversion element (a photodiode). The image generated by the imaging device 30 is output to the main body 4.

The light guide 31 is disposed in the insertion unit 3 and the main body 4. Illumination light is generated by the main body 4 and is output to the optical adapter 2 via the light guide 31. The illumination light is incident on the illumination lens unit 21 of the optical adapter 2 and is emitted to the inside of a pipe from the illumination lens unit 21.

The sensor 32 is fixed to the distal end portion 34 and outputs sensor data indicating a gravitational direction in the distal end portion 34. For example, the sensor 32 is a three-axis acceleration sensor. The sensor 32 may be a combination of an acceleration sensor and another sensor. For example, the sensor 32 includes an acceleration sensor and includes at least one of a gyro sensor and a geomagnetic sensor.

The bending portion 33 bends the insertion unit 3 upward (U), downward (D), leftward (L), or rightward (R). Alternatively, the bending portion 33 bends the insertion unit 3 up-leftward (UL), up-rightward (UR), down-leftward (DL), or down-rightward (DR). Each direction indicates a relative direction with respect to the distal end portion 34.

The main body 4 includes an image processing unit 40, a light source 41, a light source control unit 42, a motor unit 43, a bending control unit 44, a display 45, a memory unit 46, an operation unit 47, a bending joystick 48, an A/D converter 49, and a control unit 50.

The image processing unit 40 converts a format of an image output from the imaging device 30 and outputs the image to the control unit 50.

The light source 41 is a light-emitting diode (LED) or the like and generates illumination light. The illumination light is output from the light source 41 to the light guide 31. The light source control unit 42 controls the light source 41.

The motor unit 43 includes a first motor and a second motor. The first motor is connected to a wire W1 for bending the bending portion 33 in the U direction or the D direction. The second motor is connected to a wire W2 for bending the bending portion 33 in the R direction or the L direction. The wire W1 and the wire W2 are connected to the bending portion 33. The first motor bends the bending portion 33 in the U direction or the D direction by pulling the wire W1. The second motor bends the bending portion 33 in the R direction or the L direction by pulling the wire W2.

The bending control unit 44 controls a bending state of the insertion unit 3 by controlling driving of the motor unit 43.

The display 45 is a monitor such as a liquid crystal display (LCD). The display 45 displays an image generated by the imaging device 30.

The memory unit 46 includes a volatile memory and a nonvolatile memory. The volatile memory is a random-access memory (RAM), a dynamic RAM (DRAM), or the like. The volatile memory stores various types of information processed by the control unit 50. The nonvolatile memory is a static RAM (SRAM), an erasable programmable read-only memory (EPROM), an electrically erasable programmable read only-memory (EEPROM), or a flash memory. The nonvolatile memory may be detachably attached to the main body 4. The nonvolatile memory stores an image generated by the imaging device 30 and various kinds of information processed by the control unit 50.

The operation unit 47 includes a button and a touch panel for receiving various instructions from a user. The user can input various instructions to the endoscope system 1 by operating the operation unit 47. The touch panel is provided on the screen of the display 45. The user can input an instruction to change settings of the endoscope system 1, an instruction required for operating the endoscope system 1, and the like to the endoscope system 1 by operating the touch panel.

The bending joystick 48 is a rod-shaped movable member and serves as a physical user interface. The user applies a force to the bending joystick 48 by operating the bending joystick 48 with the finger or the like. Accordingly, the user can tilt the bending joystick 48 upward, downward, leftward, or rightward. The user can bend the insertion unit 3 by tilting the bending joystick 48 in a predetermined direction. The bending joystick 48 outputs an analog voltage corresponding to a direction in which the bending joystick 48 is tilted and an angle at which the bending joystick 48 is tilted.

The user may press the bending joystick 48. When the bending joystick 48 is pressed, the bending joystick 48 outputs an analog voltage. When the bending joystick 48 is pressed, a bending locking process described later is executed. While the user is tilting the bending joystick 48, a bending angle of the insertion unit 3 increases. When the bending angle of the insertion unit 3 reaches a maximum angle, the bending angle of the insertion unit 3 does not increase any more. When the bending locking process is executed, the bending angle of the insertion unit 3 is fixed even in a state in which the user detaches the finger from the bending joystick 48.

The A/D converter 49 converts the analog voltage output from the bending joystick 48 into a digital value and outputs the digital value to the control unit 50. The bending joystick 48 may include the A/D converter 49.

The control unit 50 controls each unit of the main body 4 and executes various processes. For example, the control unit 50 executes a coloring process, a noise reduction process, an outline emphasis process, or the like on an image output from the image processing unit 40. The control unit 50 stores the image in the memory unit 46. The image stored in the memory unit 46 may be a still image or a video. The control unit 50 superimposes information for displaying a graphical user interface (GUI) such as a menu on the image. The control unit 50 executes image processing such as color space conversion and gamma correction in accordance with the specifications of the display 45 and outputs the image to the display 45.

The control unit 50 determines a gravitational direction in the distal end portion 34 based on the sensor data output from the sensor 32. The control unit 50 outputs a control signal for bending the insertion unit 3 in a direction opposite to the gravitational direction to the bending control unit 44. The bending control unit 44 drives the motor unit 43 in accordance with the control signal output from the control unit 50. When the insertion unit 3 is bent in the direction opposite to the gravitational direction, the distal end portion 34 floats above the bottom surface of the pipe. Accordingly, the endoscope system 1 can avoid foreign matter such as sediment or oil on the bottom surface of the pipe coming into contact with the optical adapter 2, particularly, the imaging lens unit 20 or the illumination lens unit 21. When the user twists the insertion unit 3, the insertion unit 3 is bent in the direction opposite to the gravitational direction.

The endoscope system 1 operates in any one of two or more modes. The mode set in the endoscope system 1 can be switched. For example, the two or more modes include an automatic bending mode and a manual bending mode. When the automatic bending mode is set in the endoscope system 1, the control unit 50 executes a process of bending the insertion unit 3 in the direction opposite to the gravitational direction. When the manual bending mode is set in the endoscope system 1, the control unit 50 executes a process of bending the insertion unit 3 in a direction in which the bending joystick 48 is tilted based on the digital value output from the A/D converter 49.

The user inputs an instruction to set a mode to the endoscope system 1 by operating the button, the touch panel, or the like of the operation unit 47. The operation unit 47 outputs a mode signal indicating the mode to the control unit 50. The control unit 50 sets the automatic bending mode or the manual bending mode in the endoscope system 1 based on the mode signal output from the operation unit 47. The memory unit 46 stores the automatic bending mode or the manual bending mode set in the endoscope system 1.

The user may input an instruction to bend the insertion unit 3 to the endoscope system 1 by operating the touch panel of the operation unit 47. The operation unit 47 may output a signal indicating the instruction to the control unit 50. The control unit 50 may control the bending control unit 44 based on the signal output from the operation unit 47.

The imaging device 30 consecutively executes imaging and sequentially generates two or more live images. The image processing unit 40 sequentially processes the two or more live images. The control unit 50 sequentially acquires the two or more live images processed by the image processing unit 40 and executes the image processing described above. The control unit 50 sequentially outputs the two or more live images to the display 45. The display 45 sequentially displays the two or more live images. The endoscope system 1 can display an image of a pipe in real time.

At least one of the image processing unit 40, the light source control unit 42, the bending control unit 44, and the control unit 50 may be constituted by at least one of a processor and a logic circuit. For example, the processor is at least one of a central processing unit (CPU), a digital signal processor (DSP), and a graphics processing unit (GPU). For example, the logic circuit is at least one of an application-specific integrated circuit (ASIC) and a field-programmable gate array (FPGA). At least one of the image processing unit 40, the light source control unit 42, the bending control unit 44, and the control unit 50 may include one or more processors. At least one of the image processing unit 40, the light source control unit 42, the bending control unit 44, and the control unit 50 may include one or more logic circuits.

A computer of the endoscope system 1 may read a program and execute the read program. The program includes instructions for prescribing an operation of at least one of the image processing unit 40, the light source control unit 42, the bending control unit 44, and the control unit 50. That is, the function of at least one of the image processing unit 40, the light source control unit 42, the bending control unit 44, and the control unit 50 may be realized by software.

The program may be supplied, for example, using a "computer-readable recording medium" such as a flash memory. The program may be transmitted from a computer storing the program to the endoscope system 1 via a transmission medium or using carrier waves in the transmission medium. The "transmission medium" for transmitting a program is a medium having a function of transmitting information. The medium having a function of transmitting information includes a network (a communication network) such as the Internet and a communication circuit line (a communication line) such as a telephone line. The program may realize some of the functions described above. The program may be a differential file (a differential program). The functions described above may be realized in combination of the differential program with a program recorded in advance in the computer.

After the endoscope system 1 has been powered on, a user inserts the distal end portion 34 of the insertion unit 3 into a pipe. In the following example, the insertion unit 3 is inserted into a straight pipe. The pipe into which the insertion unit 3 is inserted may be disposed to be horizontal or may be tilted with respect to a horizontal plane. The user stops the distal end portion 34 in a state in which the distal end portion 34 is contact with the bottom surface of the pipe at a place without foreign matter. At this time, the distal end portion 34 is parallel to the bottom surface of the pipe. Thereafter, the user causes the distal end portion 34 to advance inwardly in the pipe. In the following example, a direct-view optical adapter is used as the optical adapter 2.

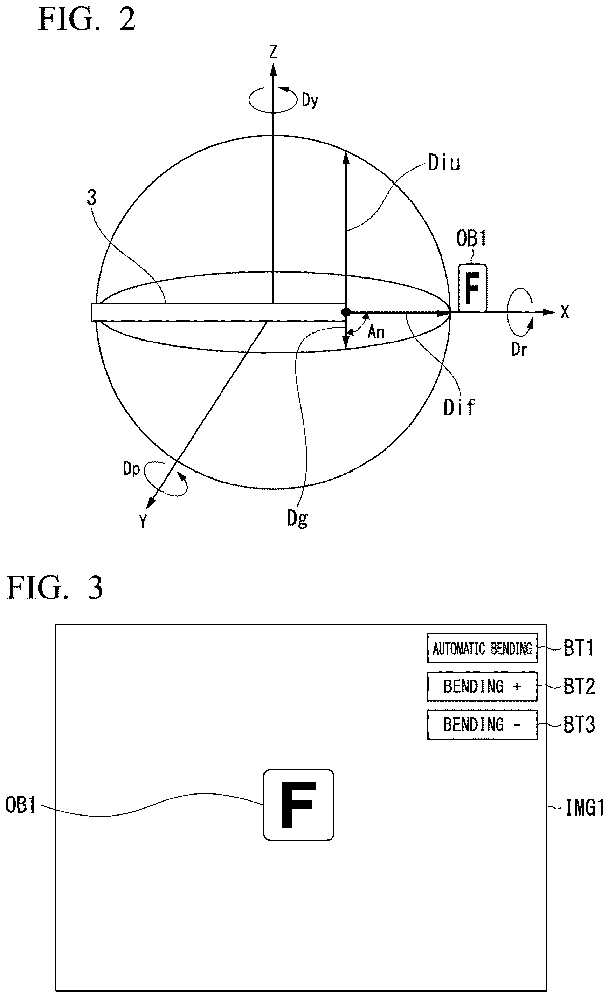

FIG. 2 shows an example of a state of the insertion unit 3 inserted into a pipe. Three-dimensional coordinate axes, that is, an X axis, a Y axis, and a Z axis orthogonal to each other, are shown. A roll direction Dr around the X axis, a pitch direction Dp around the Y axis, and a yaw direction Dy around the Z axis are shown.

The XY plane is the same as the horizontal plane. The positive direction of the Z axis is the same as a vertically upward direction. The pipe into which the insertion unit 3 is inserted is disposed to be horizontal. The axial direction of the pipe is parallel to the X axis. The insertion unit 3 in the pipe is not bent. A forward direction Dif of the distal end portion 34 and the optical adapter 2 is parallel to the X axis. The forward direction Dif matches an optical axis direction of one or more lenses of the imaging lens unit 20. An upward direction Diu corresponds to the upward direction in an image generated by the imaging device 30 and is parallel to the Z axis. A gravitational direction Dg is opposite to the upward direction Diu. At this time, an angle An between the forward direction Dif and the gravitational direction Dg is 90 degrees. In consideration of a case in which the pipe is not disposed to be horizontal, the angle An is, for example, greater than or equal to 45 degrees and less than or equal to 135 degrees.

A virtual object OB1 that is not present in an actual pipe is disposed at the center of the pipe. The virtual object OB1 is disposed in front of the distal end portion 34 and the optical adapter 2. In order to easily understand a tilt of the virtual object OB1 in an image generated by the imaging device 30, a character F is displayed on the surface of the virtual object OB1.

FIG. 3 shows an example of an image displayed on the display 45 when the insertion unit 3 is in the state shown in FIG. 2. An image IMG1 shown in FIG. 3 includes an automatic bending button BT1, a bending + button BT2, and a bending - button BT3. The virtual object OB1 appears at the center of the image IMG1. The upward direction in the image IMG1 matches the upward direction Diu in FIG. 2.

The control unit 50 superimposes the automatic bending button BT1, the bending + button BT2, and the bending - button BT3 on the image IMG1. These buttons are GUIs. The control unit 50 outputs the image IMG1 on which the buttons are superimposed to the display 45. The display 45 displays the image IMG1 on which the buttons are superimposed.

The automatic bending button BT1 is used to set the automatic bending mode in the endoscope system 1. The automatic bending button BT1 is also used to release setting of the automatic bending mode and to set the manual bending mode in the endoscope system 1. The bending + button BT2 is used to increase the angle An in the automatic bending mode. The bending - button BT3 is used to decrease the angle An in the automatic bending mode.

A user can press each button by pressing a position on the touch panel corresponding to the button. When each button is pressed, the operation unit 47 outputs a signal corresponding to the function of the button to the control unit 50.

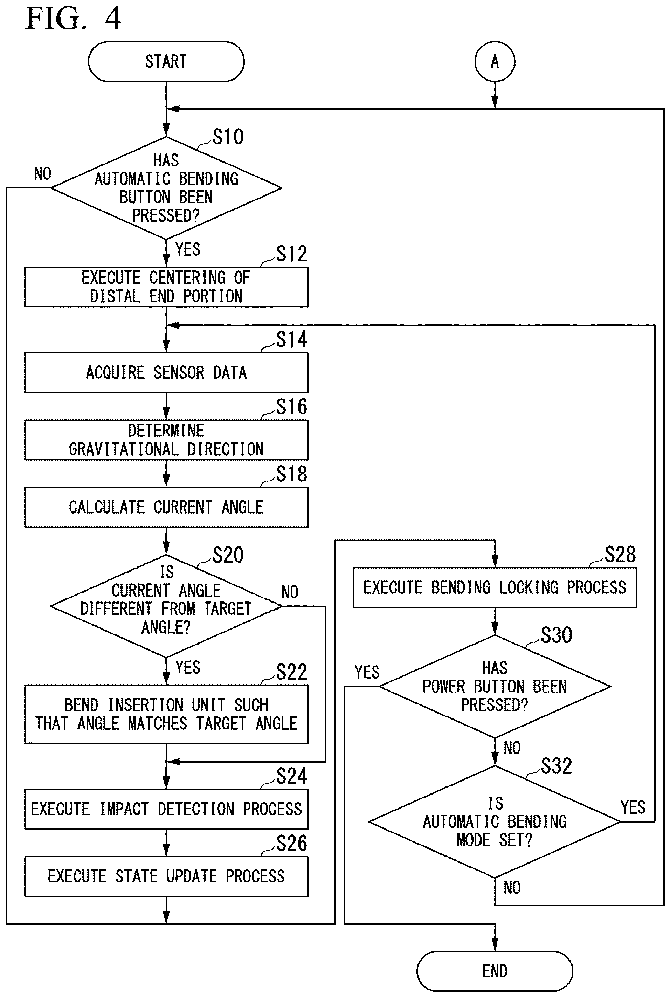

FIG. 4 shows an example of a procedure of a bending control process executed by the endoscope system 1. The operation of the endoscope system 1 in the bending control process will be described with reference to FIG. 4.

Immediately after the endoscope system 1 has been activated, the mode in the endoscope system 1 is set to the manual bending mode. The control unit 50 monitors a signal output from the operation unit 47 and determines whether the automatic bending button BT1 has been pressed (Step S10).

A user inserts the insertion unit 3 into a pipe while viewing an image displayed on the display 45. When the insertion unit 3 reaches a place at which foreign matter is assumed to be present, the user may press the automatic bending button BT1. The user can set the mode of the endoscope system 1 to the automatic bending mode at a desired timing.

When the automatic bending button BT1 is pressed, the operation unit 47 outputs a mode signal indicating the automatic bending mode to the control unit 50. When the mode signal indicating the automatic bending mode is output from the operation unit 47, the control unit 50 determines that the automatic bending button BT1 has been pressed. At this time, the control unit 50 sets the mode of the endoscope system 1 to the automatic bending mode. When the mode signal indicating the automatic bending mode is not output from the operation unit 47, the control unit 50 determines that the automatic bending button BT1 has not been pressed.

When the control unit 50 determines that the automatic bending button BT1 has not been pressed in Step S10, Step S28 described later is executed. When the control unit 50 determines that the automatic bending button BT1 has been pressed in Step S10, the control unit 50 executes centering of the distal end portion 34. At this time, the control unit 50 outputs a control signal for forcibly straightening the insertion unit 3 to the bending control unit 44. The bending control unit 44 controls the motor unit 43 such that the insertion unit 3 is straightened (Step S12).

The sensor 32 periodically outputs sensor data. The control unit 50 acquires the sensor data output from the sensor 32 and stores the sensor data in the memory unit 46. The control unit 50 repeats this process.

After Step S12, the control unit 50 acquires sensor data from the memory unit 46 (Step S14).

After Step S14, the control unit 50 determines the gravitational direction using the sensor data. The control unit 50 can determine the gravitational direction in a coordinate system specific to the distal end portion 34 (Step S16).

After Step S14, the control unit 50 calculates the current angle An based on the gravitational direction determined in Step S14. The relative positional relationship between the imaging device 30 and the sensor 32 is designed in advance and is known. The control unit 50 can calculate the angle An based on the positional relationship (Step S18).

The memory unit 46 stores a target angle in the automatic bending mode in advance. The target angle is a predetermined value of the angle An. The target angle is greater than 90 degrees and less than or equal to 180 degrees. For example, the target angle is 110 degrees. After Step S18, the control unit 50 acquires the target angle from the memory unit 46. The control unit 50 determines whether the angle An calculated in Step S18 is different from the target angle (Step S20).

In this example, the target angle indicates a target value of the angle An. The memory unit 46 may store a target value of the amount of increase of the angle An. For example, the target value may be 20 degrees. The control unit 50 may calculate the target angle by adding 20 degrees to 90 degrees that is the initial value of the angle An.

The memory unit 46 may store two or more different target angles. Each of the two or more target angles may correspond to the type of the optical adapter 2. For example, the memory unit 46 may store a target angle of a direct-view optical adapter and a target angle of a side-view optical adapter. The control unit 50 may acquire the target angle corresponding to the type of the optical adapter 2 from the memory unit 46.

The user may input information indicating the type of the optical adapter 2 to the endoscope system 1 by operating the operation unit 47. The control unit 50 may acquire the target angle corresponding to the type indicated by the information input by the user from the memory unit 46.

The optical adapter 2 may include a storage unit for storing an identifier corresponding to the type of the optical adapter 2. The control unit 50 may detect the identifier stored in the storage unit and identify the type of the optical adapter 2 corresponding to the identifier. The control unit 50 may acquire the target angle corresponding to the identified type from the memory unit 46.

A range in which the insertion unit 3 is bendable changes in accordance with the diameter of the pipe into which the insertion unit 3 is inserted. Accordingly, the two or more target angles stored in the memory unit 46 may correspond to the diameter of the pipe.

When the control unit 50 determines that the angle An is the same as the target angle in Step S20, Step S24 described later is executed. When the control unit 50 determines that the angle An is different from the target angle in Step S20, the control unit 50 outputs a control signal for bending the insertion unit 3 in the direction opposite to the gravitational direction such that the angle An matches the target angle to the bending control unit 44. The bending control unit 44 controls the motor unit 43 in accordance with the control signal and bends the insertion unit 3 in the direction opposite to the gravitational direction such that the angle An matches the target angle (Step S22).

In a state in which the angle An matches the target angle, the optical adapter 2 does not come into contact with the bottom surface of the pipe. Accordingly, foreign matter on the bottom surface of the pipe does not come into contact with the optical adapter 2.

When the user twists the insertion unit 3, the relative gravitational direction in the distal end portion 34 changes. In a condition in which the bending state of the insertion unit 3 is fixed, the angle An departs from the target angle in accordance with the amount of twist of the insertion unit 3. Accordingly, the control unit 50 outputs a control signal (a state control signal) for bending the insertion unit 3 such that the angle An is maintained at the target angle to the bending control unit 44.

After the insertion unit 3 has been bent such that the angle An matches the target angle, the control unit 50 outputs a control signal for controlling the bending state of the insertion unit 3 such that the angle An is fixed to the target angle to the bending control unit 44. The bending control unit 44 controls the motor unit 43 in accordance with the control signal and fixes the angle An to the target angle. While the mode of the endoscope system 1 is set to the automatic bending mode, the control unit 50 continues to execute the control.

The memory unit 46 may store a range of the target angle. The control unit 50 may determine whether the angle An is included in the range of the target angle in Step S20. When the control unit 50 determines that the angle An is not included in the range of the target angle in Step S20, Step S24 may be executed. When the control unit 50 determines that the angle An is included in the range of the target angle in Step S20, Step S22 may be executed. In Step S22, the control unit 50 may output a control signal for bending the insertion unit 3 in the direction opposite to the gravitational direction such that the angle An is included in the range of the target angle to the bending control unit 44.

After Step S22, the control unit 50 executes an impact detection process of detecting a strong impact in the distal end portion 34 (Step S24). Details of the impact detection process will be described later. The bending control process need not include the impact detection process.

After Step S24, the control unit 50 executes a state update process of controlling the bending state of the insertion unit 3 in accordance with the user's operation of the bending + button BT2 or the bending - button BT3 (Step S26). Details of the state update process will be described later. The bending control process need not include the state update process.

After Step S26, the control unit 50 executes a bending locking process of fixing the bending state of the insertion unit 3 (Step S28). Details of the bending locking process will be described later. The bending control process need not include the bending locking process.

After Step S28, the control unit 50 monitors the signal output from the operation unit 47 and determines whether a power button of the operation unit 47 has been pressed (Step S30).

When the control unit 50 determines that the power button has been pressed in Step S30, the bending control process shown in FIG. 4 ends. When the control unit 50 determines that the power button has not been pressed in Step S30, the control unit 50 determines whether the automatic bending mode is set in the endoscope system 1 (Step S32).

When the control unit 50 determines that the automatic bending mode is set in the endoscope system 1 in Step S32, Step S14 is executed. When the control unit 50 determines that the automatic bending mode is not set in the endoscope system 1 in Step S32, Step S10 is executed. At this time, the distal end portion 34 is in contact with the bottom surface of the pipe and is parallel to the bottom surface of the pipe.

When the automatic bending mode is set in the endoscope system 1, the user can change the automatic bending mode to the manual bending mode by pressing the automatic bending button BT1. When the automatic bending button BT1 has been pressed, the operation unit 47 outputs the mode signal indicating the manual bending mode to the control unit 50. When the mode signal indicating the manual bending mode is output from the operation unit 47, the control unit 50 sets the mode of the endoscope system 1 to the manual bending mode.

The target angle stored in the memory unit 46 may be changeable. For example, the user may input an arbitrary value of the target angle to the endoscope system 1 by operating the operation unit 47. The control unit 50 may change the target angle stored in the memory unit 46 to an angle corresponding to the value input to the endoscope system 1.

The control unit 50 may output a GUI image including two or more values of the target angle to the display 45. The user may input an instruction to select one of the two or more values to the endoscope system 1 via the GUI by operating the touch panel of the operation unit 47. The control unit 50 may change the target angle stored in the memory unit 46 to an angle corresponding to the value indicated by the instruction input to the endoscope system 1.

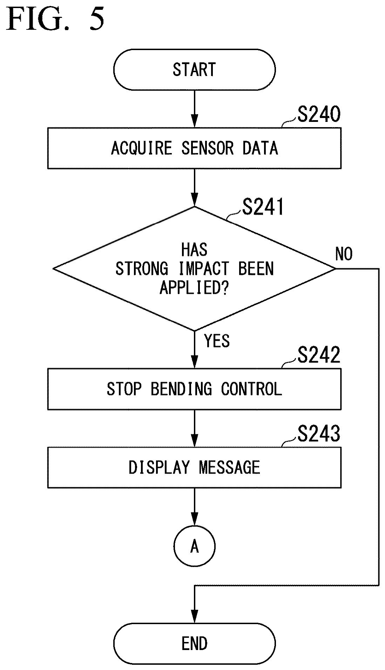

FIG. 5 shows an example of a procedure of the impact detection process. The operation of the endoscope system 1 in the impact detection process will be described with reference to FIG. 5.

The control unit 50 acquires sensor data from the memory unit 46 (Step S240).

After Step S240, the control unit 50 determines whether a strong impact has been applied to the distal end portion 34 based on the sensor data (Step S241).

In Step S241, the control unit 50 determines the acceleration in the distal end portion 34. When the acceleration is greater than a predetermined value, the control unit 50 determines that a strong impact has been applied to the distal end portion 34. When the acceleration is not greater than the predetermined value, the control unit 50 determines that a strong impact has not been applied to the distal end portion 34.

When the control unit 50 determines that a strong impact has not been applied to the distal end portion 34 in Step S241, Step S26 shown in FIG. 4 is executed. When the control unit 50 determines that a strong impact has been applied to the distal end portion 34 in Step S241, the control unit 50 stops the bending control. At this time, the control unit 50 may execute the same process as Step S12 (Step S242).

In inspection of a thin pipe, there is a likelihood that the insertion unit 3 will come into contact with an inner wall of the pipe and the bending portion 33 will be broken. Accordingly, when a strong impact has been applied to the distal end portion 34, the control unit 50 stops the bending control.

After Step S242, the control unit 50 outputs a message indicating that the bending control has been stopped to the display 45. The display 45 displays the message (Step S243). After Step S243, Step S10 shown in FIG. 4 is executed.

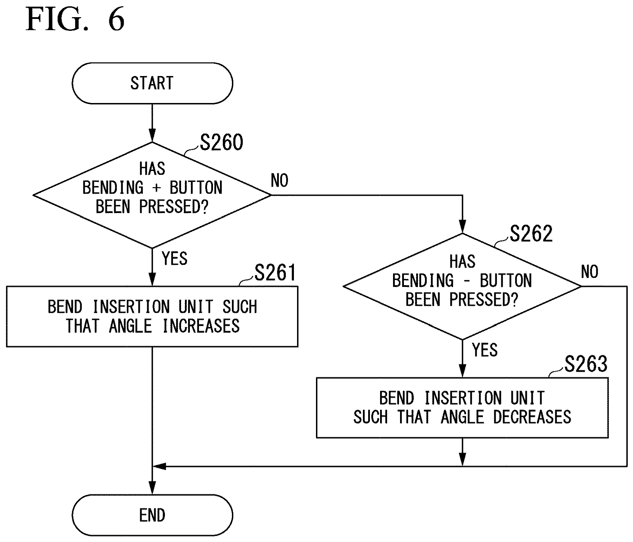

FIG. 6 shows an example of a procedure of the state update process. The operation of the endoscope system 1 in the state update process will be described with reference to FIG. 6.

The memory unit 46 stores a set value of the amount of increase of the angle An and a set value of the amount of decrease of the angle An in advance. The memory unit 46 may store two or more set values of the amount of increase of the angle An and two or more set values of the amount of decrease of the angle An in advance. Each of the two or more set values of the amount of increase of the angle An may correspond to the type of the optical adapter 2. Each of the two or more set values of the amount of decrease of the angle An may correspond to the type of the optical adapter 2.

The control unit 50 monitors a signal output from the operation unit 47 and determines whether the bending + button BT2 has been pressed (Step S260).

When the control unit 50 determines that the bending + button BT2 has been pressed in Step S260, the control unit 50 acquires the set value of the amount of increase of the angle An from the memory unit 46. The control unit 50 outputs a control signal for increasing the amount of bending of the insertion unit 3 such that the angle An increases by the set value to the bending control unit 44. The bending control unit 44 controls the motor unit 43 in accordance with the control signal and bends the insertion unit 3 such that the angle An increases by the set value. For example, the bending state of the insertion unit 3 is changed such that the angle An increases by 5 degrees (Step S261). After Step S261, Step S28 shown in FIG. 4 is executed.

When the control unit 50 determines that the bending + button BT2 has not been pressed in Step S260, the control unit 50 determines whether the bending - button BT3 has been pressed (Step S262).

When the control unit 50 determines that the bending - button BT3 has not been pressed in Step S262, Step S28 shown in FIG. 4 is executed. When the control unit 50 determines that the bending - button BT3 has been pressed in Step S262, the control unit 50 acquires the set value of the amount of decrease of the angle An from the memory unit 46. The control unit 50 outputs a control signal for decreasing the amount of bending of the insertion unit 3 such that the angle An decreases by the set value to the bending control unit 44. The bending control unit 44 controls the motor unit 43 in accordance with the control signal and bends the insertion unit 3 such that the angle An decreases by the set value. For example, the bending state of the insertion unit 3 is changed such that the angle An decreases by 5 degrees (Step S263). After Step S263, Step S28 shown in FIG. 4 is executed.

The set value of the amount of increase or the amount of decrease of the angle An stored in the memory unit 46 may be changeable. For example, the user may input an arbitrary set value to the endoscope system 1 by operating the operation unit 47. The control unit 50 may change the set value stored in the memory unit 46 to the set value input to the endoscope system 1.

The control unit 50 may output a GUI image including two or more set values to the display 45. The user may input an instruction to select one of the two or more values via the GUI to the endoscope system 1 by operating the touch panel of the operation unit 47. The control unit 50 may change the set values stored in the memory unit 46 to a set value indicated by the instruction input to the endoscope system 1.

The degree of tilt of the pipe with respect to the horizontal plane may change in accordance with positions. In a state in which the automatic bending mode is set in the endoscope system 1, the angle An is maintained at the target angle regardless of the degree of tilt. Accordingly, in a state in which the degree of tilt of the pipe with respect to the horizontal plane increases gradually in the vertically upward direction with advancement of the insertion unit 3, there is a likelihood that the optical adapter 2 will come into contact with the bottom surface of the pipe. The user can adjust the bending state of the insertion unit 3 such that the optical adapter 2 does not come into contact with the bottom surface of the pipe by using the bending - button BT3.

In a state in which the degree of tilt of the pipe with respect to the horizontal plane increases gradually in the vertically downward direction with advancement of the insertion unit 3, there is a likelihood that the optical adapter 2 will come into contact with the top surface of the pipe. The user can adjust the bending state of the insertion unit 3 such that the optical adapter 2 does not come into contact with the top surface of the pipe by using the bending + button BT2.

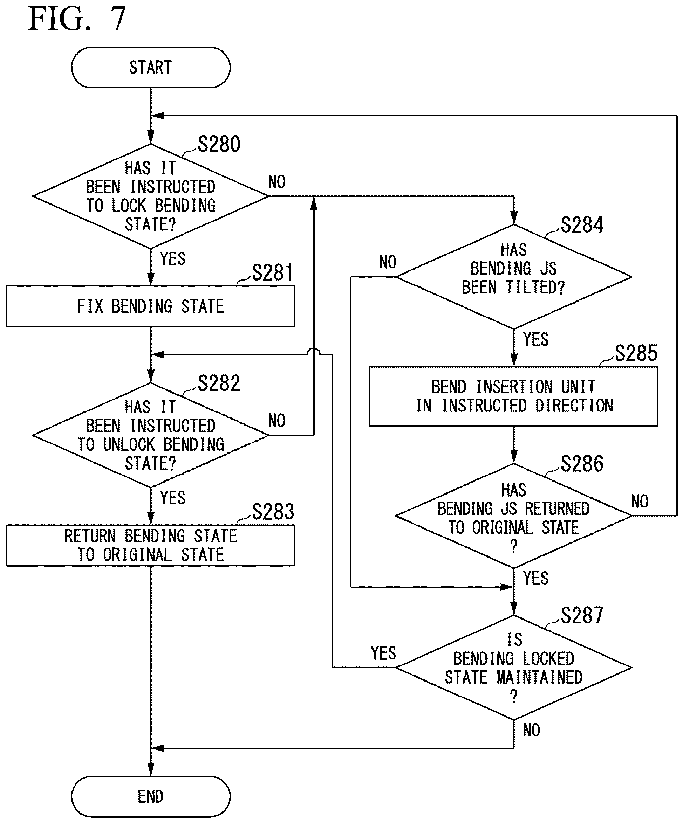

FIG. 7 shows an example of a procedure of the bending locking process. The operation of the endoscope system 1 in the bending locking process will be described with reference to FIG. 7.

The user can input an instruction to lock the bending state to the endoscope system 1 by pressing the bending joystick 48. When the bending joystick 48 has been pressed, the bending joystick 48 outputs a digital value to the control unit 50.

The control unit 50 monitors the digital value output from the bending joystick 48 and determines whether it has been instructed to lock the bending state (Step S280).

When the control unit 50 determines that it has been instructed to lock the bending state in Step S280, the control unit 50 outputs a control signal for controlling the bending state of the insertion unit 3 such that the bending state of the insertion unit 3 is fixed to the current state to the bending control unit 44. The bending control unit 44 controls the motor unit 43 in accordance with the control signal and fixes the bending state of the insertion unit 3. Until it is instructed to unlock the bending state, the control unit 50 continues to execute the control (Step S281).

After the instruction to lock the bending state has been input, the user can input an instruction to unlock the bending state to the endoscope system 1 by pressing the bending joystick 48 again. When the bending joystick 48 has been pressed, the bending joystick 48 outputs a digital value to the control unit 50.

After Step S281, the control unit 50 monitors the digital value output from the bending joystick 48 and determines whether it has been instructed to unlock the bending state (Step S282).

When the control unit 50 determines that it has been instructed to unlock the bending state in Step S282, the control unit 50 outputs a control signal for controlling the bending state of the insertion unit 3 such that the bending state of the insertion unit 3 returns to the original state to the bending control unit 44. The bending control unit 44 controls the motor unit 43 in accordance with the control signal and returns the bending state of the insertion unit 3 to the original state (Step S283). After Step S283, Step S30 shown in FIG. 4 is executed.

When Step S283 is executed, the bending state of the insertion unit 3 returns to a state before the bending joystick 48 has been tilted. When Step S283 is executed in a state in which the mode of the endoscope system 1 is set to the automatic bending mode, the bending state of the insertion unit 3 is changed such that the angle An matches the target angle.

When the control unit 50 determines that it has not been instructed to lock the bending state in Step S280, the control unit 50 monitors the digital value output from the A/D converter 49 and determines whether the bending joystick 48 has been tilted by the user (Step S284).

When the control unit 50 determines that the bending joystick 48 has not been tilted by the user in Step S284, Step S287 described later is executed. When the control unit 50 determines that the bending joystick 48 has been tilted by the user in Step S284, the control unit 50 outputs a control signal for controlling the bending state of the insertion unit 3 such that the insertion unit 3 is bent in a direction corresponding to the direction in which the bending joystick 48 has been tilted to the bending control unit 44. The bending control unit 44 controls the motor unit 43 in accordance with the control signal and bends the insertion unit 3 (Step S285).

The memory unit 46 stores a set value indicating the amount of bending by which the insertion unit 3 is bent when the bending joystick 48 has been tilted by the user in advance. The control unit 50 outputs a control signal for controlling the bending state of the insertion unit 3 such that the insertion unit 3 is bent by the amount corresponding to the set value to the bending control unit 44 in Step S285.

After Step S285, the control unit 50 monitors the digital value output from the A/D converter 49 and determines whether the bending joystick 48 has returned to the original state. The original state is a state in which the bending joystick 48 has not been tilted (Step S286).

When the control unit 50 determines that the bending joystick 48 has not returned to the original state in Step S286, Step S280 is executed. When the control unit 50 determines that the bending joystick 48 has returned to the original state in Step S286, the control unit 50 determines whether the state in which it has been instructed to lock the bending state (the bending locked state) is maintained (Step S287).

When the control unit 50 determines that it has not been instructed to lock the bending state in Step S280, the bending locked state is not maintained. When the control unit 50 determines that it has not been instructed to unlock the bending state in Step S282, the bending locked state is maintained.

When the control unit 50 determines that the bending locked state is not maintained in Step S287, Step S30 shown in FIG. 4 is executed. When the control unit 50 determines that the bending locked state is maintained in Step S287, Step S282 is executed.

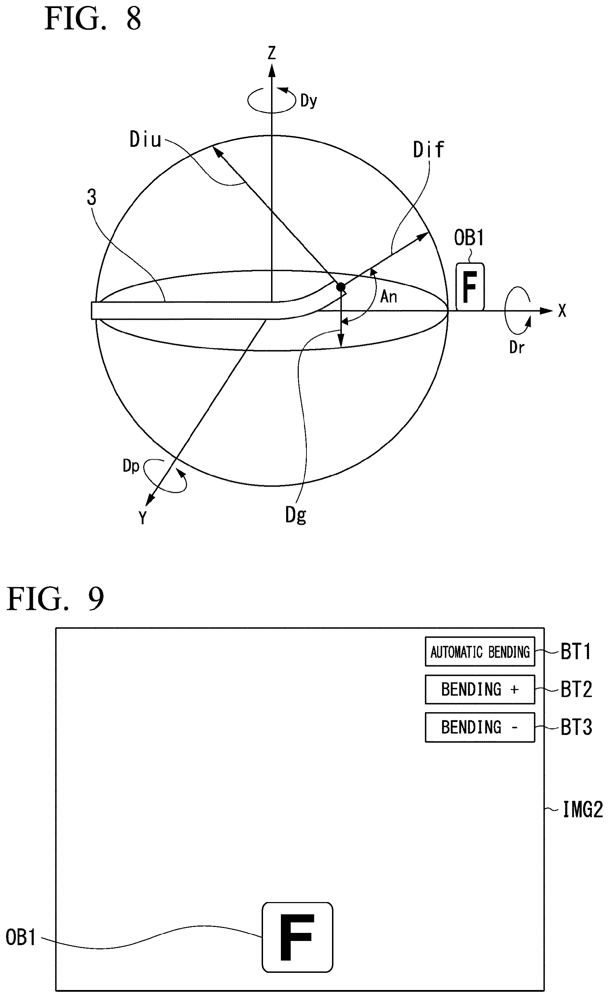

FIG. 8 shows an example of the state of the insertion unit 3 after Step S22 shown in FIG. 4 has been executed. The same parts as those shown in FIG. 2 will not be described.

The control unit 50 outputs a control signal for bending the insertion unit 3 such that the angle An matches the target angle to the bending control unit 44. The bending control unit 44 controls the motor unit 43 in accordance with the control signal and bends the insertion unit 3 such that the angle An matches the target angle. In the example shown in FIG. 8, the target angle is 110 degrees. In the example shown in FIG. 8, a user has not twisted the insertion unit 3. The insertion unit 3 is bent by 20 degrees in the vertically upward direction.

FIG. 9 shows an example of an image displayed on the display 45 when the insertion unit 3 is in the state shown in FIG. 8. An image IMG2 shown in FIG. 9 includes an automatic bending button BT1, a bending + button BT2, and a bending - button BT3. The virtual object OB1 appears in the image IMG2. The same parts as those shown in FIG. 3 will not be described.

Since the insertion unit 3 is bent in the vertically upward direction, the distal end portion 34 moves in the vertically upward direction. The virtual object OB1 in the image IMG2 moves downward. Since the insertion unit 3 is not twisted, the virtual object OB1 in the image IMG2 does not rotate.

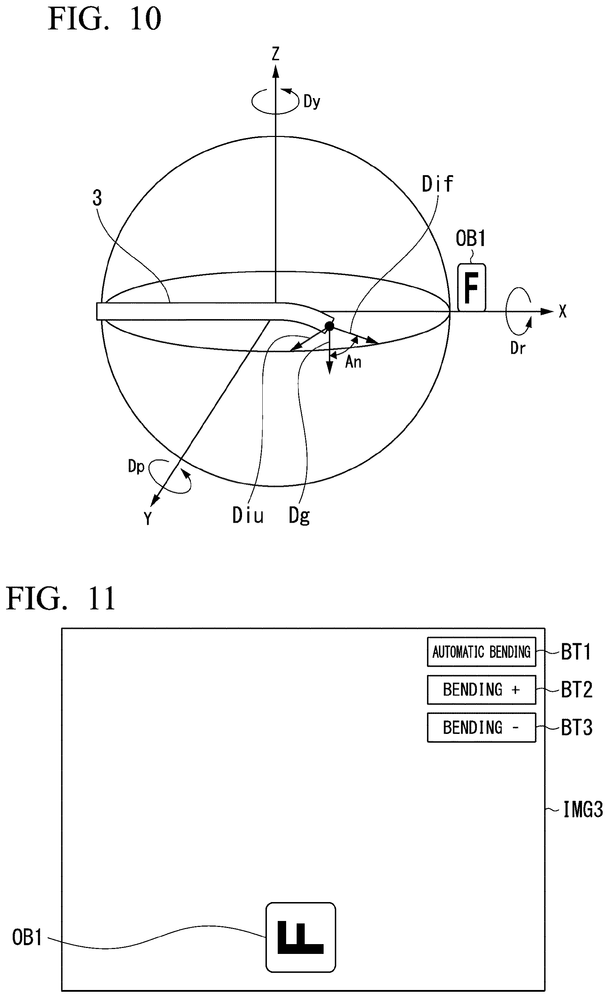

Three examples of the state of the insertion unit 3 after the insertion unit 3 has entered the state shown in FIG. 8 will be described. In a first example, the mode of the endoscope system 1 is set to the manual bending mode, and it is instructed to lock the bending state. Thereafter, the user twists the insertion unit 3 by 90 degrees in the roll direction Dr. FIG. 10 shows an example of the state of the insertion unit 3 after the insertion unit 3 has been twisted by 90 degrees in the roll direction Dr. The same parts as those shown in FIG. 2 will not be described.

While the insertion unit 3 is rotating in the roll direction Dr, the bending state of the insertion unit 3 is maintained. In a state in which the insertion unit 3 has been twisted by 90 degrees in the roll direction Dr, the distal end portion 34 comes into contact with the bottom surface of the pipe. The forward direction Dif and the upward direction Diu are parallel to the horizontal plane, and the angle An is 90 degrees.

FIG. 11 shows an example of an image displayed on the display 45 when the insertion unit 3 is in the state shown in FIG. 10. An image IMG3 shown in FIG. 11 includes an automatic bending button BT1, a bending + button BT2, and a bending - button BT3. The virtual object OB1 appears in the image IMG3. The same parts as those shown in FIG. 3 will not be described. Since the insertion unit 3 rotates in the roll direction Dr, the virtual object OB1 in the image IMG3 rotates counterclockwise.

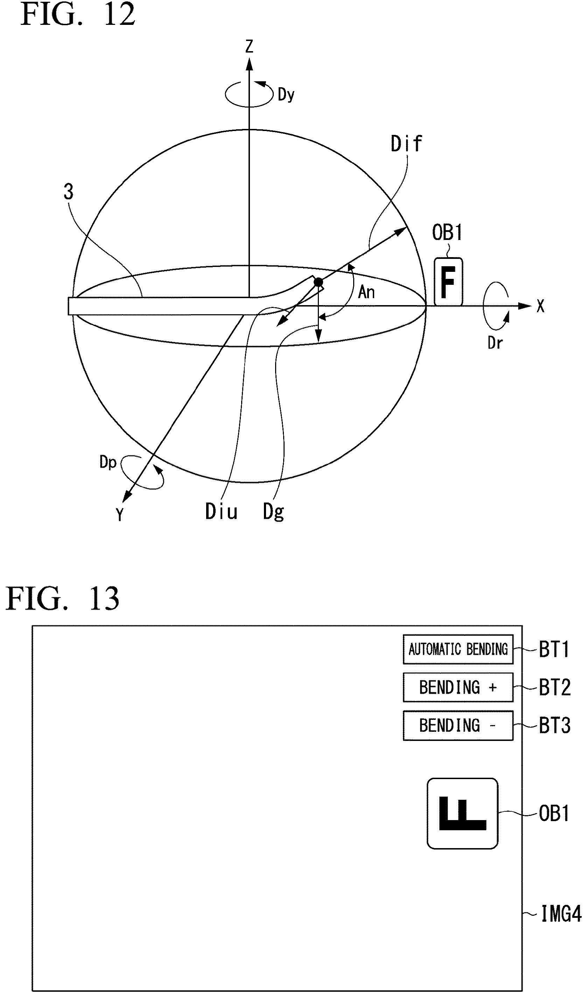

After the insertion unit 3 has entered the state shown in FIG. 8, in a second example, the mode of the endoscope system 1 is the automatic bending mode and the user twists the insertion unit 3 by 90 degrees in the roll direction Dr. FIG. 12 shows an example of the state of the insertion unit 3 after the insertion unit 3 has been twisted by 90 degrees in the roll direction Dr. The same parts as those shown in FIG. 2 will not be described.

While the insertion unit 3 is rotating in the roll direction Dr, the control unit 50 repeatedly executes Step S22 and outputs a control signal for maintaining the angle An at the target angle to the bending control unit 44. The bending control unit 44 controls the motor unit 43 in accordance with the control signal and maintains the angle An at the target angle. In the example shown in FIG. 12, the target angle is 110 degrees. Even when the insertion unit 3 is twisted, the distal end portion 34 does not come into contact with the bottom surface of the pipe.

FIG. 13 shows an example of an image displayed on the display 45 when the insertion unit 3 is in the state shown in FIG. 12. An image IMG4 shown in FIG. 13 includes an automatic bending button BT1, a bending + button BT2, and a bending - button BT3. The virtual object OB1 appears in the image IMG4. The same parts as those shown in FIG. 3 will not be described.

Since the insertion unit 3 rotates in the roll direction Dr, the virtual object OB1 in the image IMG4 rotates counterclockwise. The forward direction Dif in FIG. 12 is different from the forward direction Dif in FIG. 10. Accordingly, the position of the virtual object OB1 in the image IMG4 is different from the position of the virtual object OB1 in the image IMG3 shown in FIG. 11.

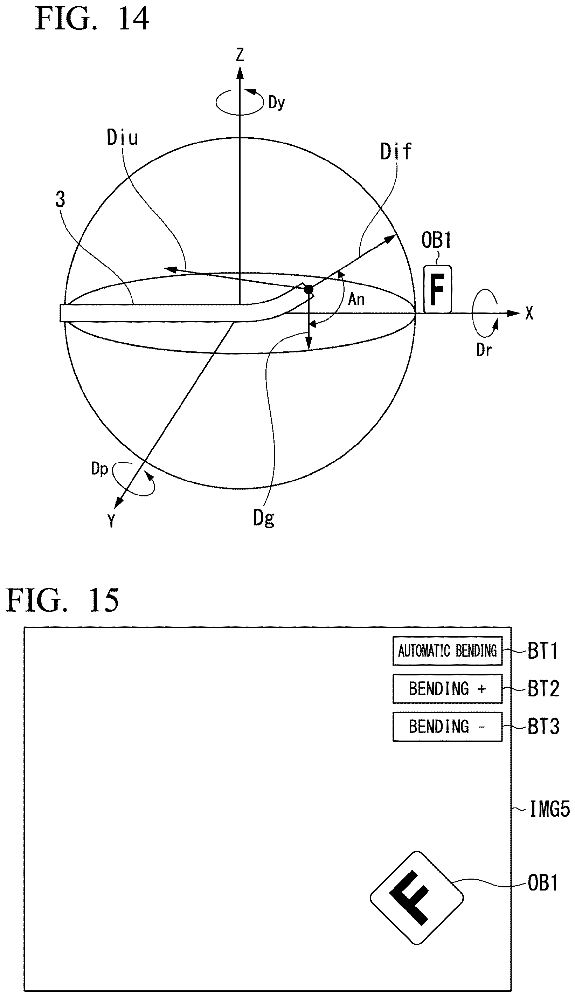

After the insertion unit 3 has entered the state shown in FIG. 8, in a third example, the mode of the endoscope system 1 is the automatic bending mode and the user twists the insertion unit 3 by 45 degrees in the roll direction Dr. FIG. 14 shows an example of the state of the insertion unit 3 after the insertion unit 3 has been twisted by 45 degrees in the roll direction Dr. The same parts as those shown in FIG. 2 will not be described.

As described above, the control unit 50 outputs the control signal for maintaining the angle An at the target angle to the bending control unit 44. The bending control unit 44 controls the motor unit 43 in accordance with the control signal and maintains the angle An at the target angle. In the example shown in FIG. 14, the target angle is 110 degrees.

FIG. 15 shows an example of an image displayed on the display 45 when the insertion unit 3 is in the state shown in FIG. 14. An image IMG5 shown in FIG. 15 includes an automatic bending button BT1, a bending + button BT2, and a bending - button BT3. The virtual object OB1 appears at the center of the image IMG5. The same parts as those shown in FIG. 3 will not be described.

In comparison with the state shown in FIG. 9, the virtual object OB1 in the image IMG5 rotates by 45 degrees counterclockwise. The forward direction Dif in FIG. 14 is different from the forward direction Dif in FIG. 10. Accordingly, the position of the virtual object OB1 in the image IMG5 is different from the position of the virtual object OB1 in the image IMG3 shown in FIG. 11.

When the insertion unit 3 is twisted in the state in which the mode of the endoscope system 1 is set to the automatic bending mode, the control unit 50 outputs a control signal for bending the insertion unit 3 in the U direction, the D direction, the R direction, or the L direction such that the angle An increases in accordance with the amount of twist of the insertion unit 3 to the bending control unit 44. The bending control unit 44 controls the first motor and the second motor in accordance with the control signal. The first motor bends the insertion unit 3 in the U direction or the D direction by pulling the wire W1. The second motor bends the insertion unit 3 in the R direction or the L direction by pulling the wire W2.

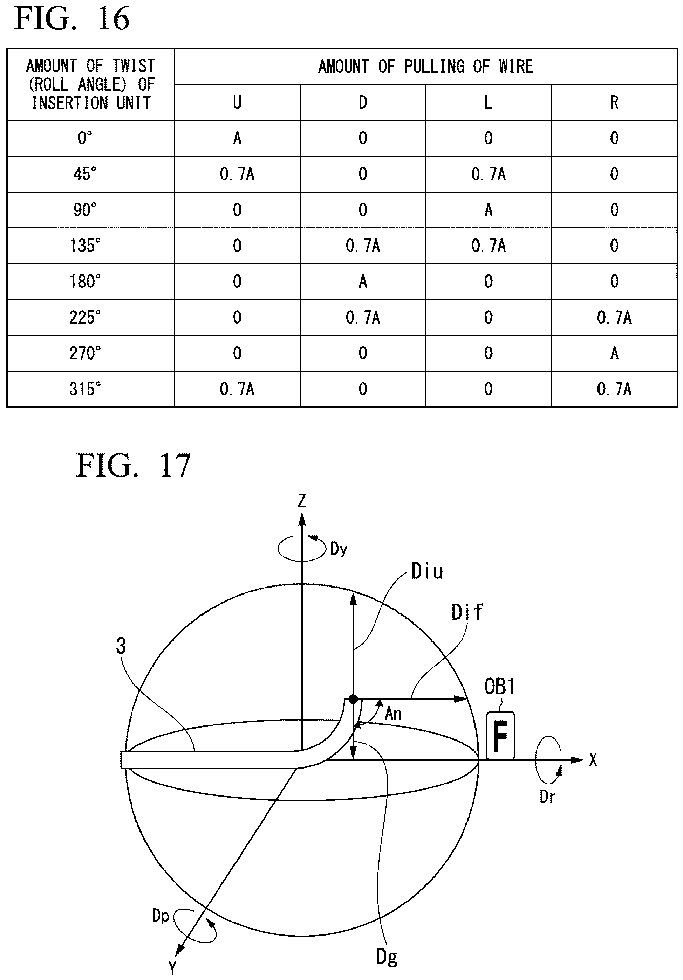

FIG. 16 shows a relationship between the amount of twist of the insertion unit 3 in the roll direction and the amount of pulling of the wire W1 and the wire W2. When the insertion unit 3 has not been twisted, it is necessary to pull the wire W1 by A in order to bend the insertion unit 3 in the U direction such that the angle An becomes 110 degrees. When the insertion unit 3 has been twisted by 45 degrees in the roll direction, it is necessary to bend the insertion unit 3 in the U direction and the L direction such that the angle An becomes 110 degrees. At this time, the amount of pulling of the wire W1 and the wire W2 is 0.7 A.

When the insertion unit 3 has been twisted by 90 degrees in the roll direction, it is necessary to pull the wire W1 by A in order to bend the insertion unit 3 in the L direction such that the angle An reaches the target angle. When the insertion unit 3 has been twisted by 135 degrees in the roll direction, it is necessary to bend the insertion unit 3 in the D direction and the L direction such that the angle An reaches 110 degrees. At this time, the amount of pulling of the wire W1 and the wire W2 is 0.7 A.

FIG. 16 shows the amount of pulling of the wire W1 and the wire W2 required for bending the insertion unit 3 when the amount of twist of the insertion unit 3 in the roll direction is 180 degrees, 225 degrees, 270 degrees, or 360 degrees. FIG. 16 shows the amount of pulling of the wire W1 and the wire W2 when a predetermined amount of twist has been applied to the insertion unit 3 in an ideal state in which the insertion unit 3 is straight. Actually, a larger amount of pulling than the amount of pulling shown in FIG. 16 may be required for maintaining the angle An at the target angle.

FIG. 17 shows the state of the insertion unit 3 after Step S22 shown in FIG. 4 has been executed in the state in which a side-view optical adapter is used as the optical adapter 2. The same parts as those shown in FIG. 2 will not be described.

The control unit 50 outputs a control signal for bending the insertion unit 3 such that the angle An matches the target angle to the bending control unit 44. The bending control unit 44 controls the motor unit 43 in accordance with the control signal and bends the insertion unit 3 such that the angle An matches the target angle. The target angle of the side-view optical adapter is different from the target angle of the direct-view optical adapter. In the example shown in FIG. 17, the target angle is 90 degrees. In the example shown in FIG. 17, the user has not twisted the insertion unit 3. The insertion unit 3 is bent by 90 degrees in the vertically upward direction. The forward direction Dif of the side-view optical adapter 2 is parallel to the X axis.

When the user twists the insertion unit 3 in the roll direction Dr, the angle An is maintained at 90 degrees, and the distal end portion 34 rotates in the yaw direction Dy. The optical axis of the imaging lens unit 20 of the optical adapter 2 rotates in the yaw direction Dy. The user can direct the optical axis of the imaging lens unit 20 in an arbitrary direction parallel to the XY plane by twisting the insertion unit 3 in the roll direction Dr.

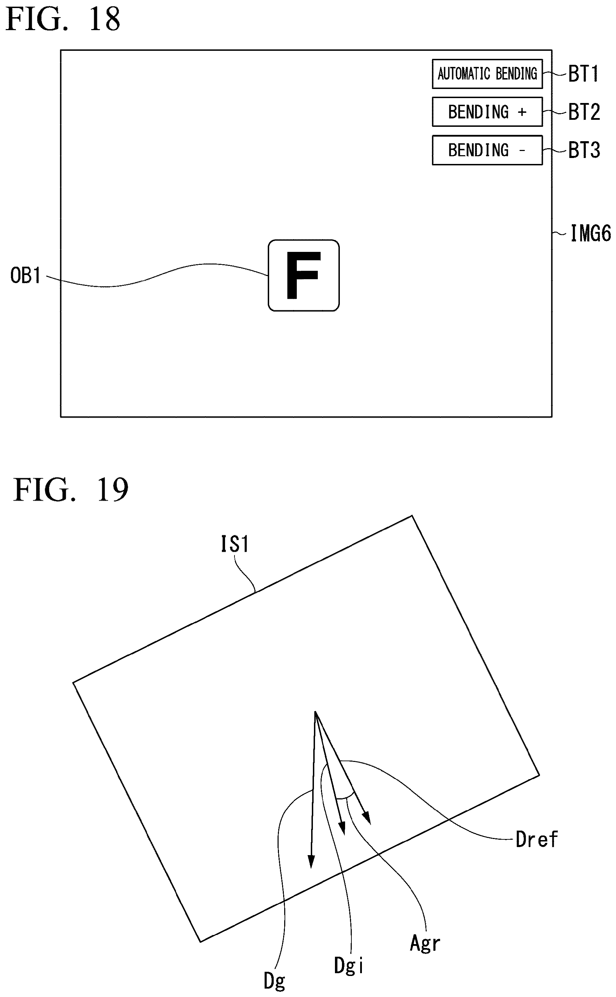

FIG. 18 shows an example of an image displayed on the display 45 when the insertion unit 3 is in the state shown in FIG. 17. An image IMG6 shown in FIG. 18 includes an automatic bending button BT1, a bending + button BT2, and a bending - button BT3. The virtual object OB1 appears in the image IMG6. The same parts as those shown in FIG. 3 will not be described.

Since the insertion unit 3 is bent in the vertically upward direction, the distal end portion 34 moves in the vertically upward direction. The virtual object OB1 is located slightly below the center of the image IMG6.

As described above, the relative positional relationship between the imaging device 30 and the sensor 32 is known. The control unit 50 may execute the following process in Step S16 shown in FIG. 4. The control unit 50 generates information indicating a relationship between the gravitational direction and a reference direction on the imaging surface of the imaging device 30. For example, the reference direction is a direction perpendicular to the imaging surface. The control unit 50 superimposes the information on the image generated by the imaging device 30 and outputs the image on which the information has been superimposed to the display 45. The display 45 displays the image.

The information indicating the relationship between the gravitational direction and the reference direction represents the posture of the distal end portion 34. The user can determine the posture of the distal end portion 34 based on the image displayed on the display 45.

The control unit 50 may execute the following process in Step S16 shown in FIG. 4. FIG. 19 shows an example of a relationship between an imaging surface IS1 of the imaging device 30 and the gravitational direction Dg.

The control unit 50 calculates a component Dgi in the gravitational direction Dg in the imaging surface IS1. The component Dgi is parallel to the imaging surface IS1. The control unit 50 calculates an angle Agr between the direction of the component Dgi and the reference direction Dref in the imaging surface IS1. The reference direction Dref is parallel to the imaging surface IS1. For example, the reference direction Dref is a downward direction in the imaging surface IS1. The downward direction in the imaging surface IS1 corresponds to the downward direction in an image. The control unit 50 corrects an image generated by the imaging device 30 by rotating the image by the angle Agr. The downward direction in the corrected image matches the direction of the component Dgi. The control unit 50 outputs the corrected image to the display 45. The display 45 displays the image.

The endoscope system 1 can display an image generated by the imaging device 30 in a state in which the direction of the component Dgi corresponding to the gravitational direction Dg matches the downward direction in the image. Since the direction of the component Dgi in the image does not change, the user can execute inspection without being confused.

According to each aspect of the present invention, the endoscope system 1 includes the insertion unit 3 and the control unit 50. The insertion unit 3 includes the sensor 32 in the distal end portion 34 and is bendable. The control unit 50 receives the mode signal indicating the mode in which the insertion unit 3 is automatically bent. The control unit 50 acquires data output from the sensor 32 and determines the gravitational direction Dg in the distal end portion 34 based on the data. After the mode signal has been received, the control unit 50 outputs a control signal for bending the insertion unit 3 such that the angle An between the forward direction Dif of the distal end portion 34 and the gravitational direction Dg increases.

According to each aspect of the present invention, the control method includes first to fourth steps. In a first step (Step S10), the control unit 50 receives the mode signal indicating the mode in which the insertion unit 3 is automatically bent. In a second step (Step S14), the control unit 50 acquires data output from the sensor 32. In a third step (Step S16), the control unit 50 determines the gravitational direction in the distal end portion 34 based on the data. In a fourth step (S22), after the mode signal has been received, the control unit 50 outputs a control signal for bending the insertion unit 3 such that the angle An between the forward direction Dif of the distal end portion 34 and the gravitational direction Dg increases.

According to each aspect of the present invention, a program causes a computer to execute the first to fourth steps.

Each aspect of the present invention may include the following modified example. After the control signal has been output, the control unit 50 outputs a state control signal for controlling the bending state of the insertion unit 3 such that the angle An is fixed.

Each aspect of the present invention may include the following modified example. The control unit 50 outputs the control signal for bending the insertion unit 3 in a first direction and a second direction perpendicular to the first direction such that the angle An increases in accordance with the amount of twist of the insertion unit 3. In the example described above, the first direction is the U direction or the D direction, and the second direction is the R direction or the L direction.

Each aspect of the present invention may include the following modified example. The control unit 50 generates the control signal based on a predetermined value stored in the memory unit 46. The predetermined value indicates a target value of the angle An or a target value of the amount of increase of the angle An.

Each aspect of the present invention may include the following modified example. The memory unit 46 stores two or more values as the predetermined value. The control unit 50 acquires any one of the two or more values from the memory unit 46.

Each aspect of the present invention may include the following modified example. The control unit 50 acquires the predetermined value corresponding to the type of the optical adapter 2 attached to the distal end portion 34 from the memory unit 46.

Each aspect of the present invention may include the following modified example. The predetermined value is changeable.

Each aspect of the present invention may include the following modified example. The sensor 32 is an acceleration sensor. The data output from the sensor 32 indicates the acceleration of the distal end portion 34.

Each aspect of the present invention may include the following modified example. The endoscope system 1 includes the imaging device 30 (an image sensor) that generates an image. The control unit 50 determines a posture of the distal end portion 34 based on the gravitational direction Dg. The control unit 50 superimposes information indicating the posture of the distal end portion 34 on the image generated by the imaging device 30 and outputs the image on which the information has been superimposed to the display 45.

Each aspect of the present invention may include the following modified example. The control unit 50 outputs an image for displaying a GUI associated with setting of the mode in which the insertion unit 3 is automatically bent to the display 45. When it is instructed to set the mode via the GUI, the control unit 50 receives the mode signal.

Each aspect of the present invention may include the following modified example. The endoscope system 1 includes the imaging device 30 (an image sensor) that generates an image. The control unit 50 calculates a component in the gravitational direction on the imaging surface of the imaging device 30. The control unit 50 calculates the angle between the direction of the component in the gravitational direction on the imaging surface and the reference direction on the imaging surface. The control unit 50 corrects an image generated by the imaging device 30 by rotating the image by the angle.

The endoscope system 1 according to the first embodiment bends the insertion unit 3 such that the angle An between the forward direction Dif of the distal end portion 34 and the gravitational direction Dg increases in the automatic bending mode. Accordingly, the endoscope system 1 does not require a user's complicated operations and reduce foreign matter in contact with the observation optical system. The user can efficiently perform inspection.

### (First modified example of first embodiment)

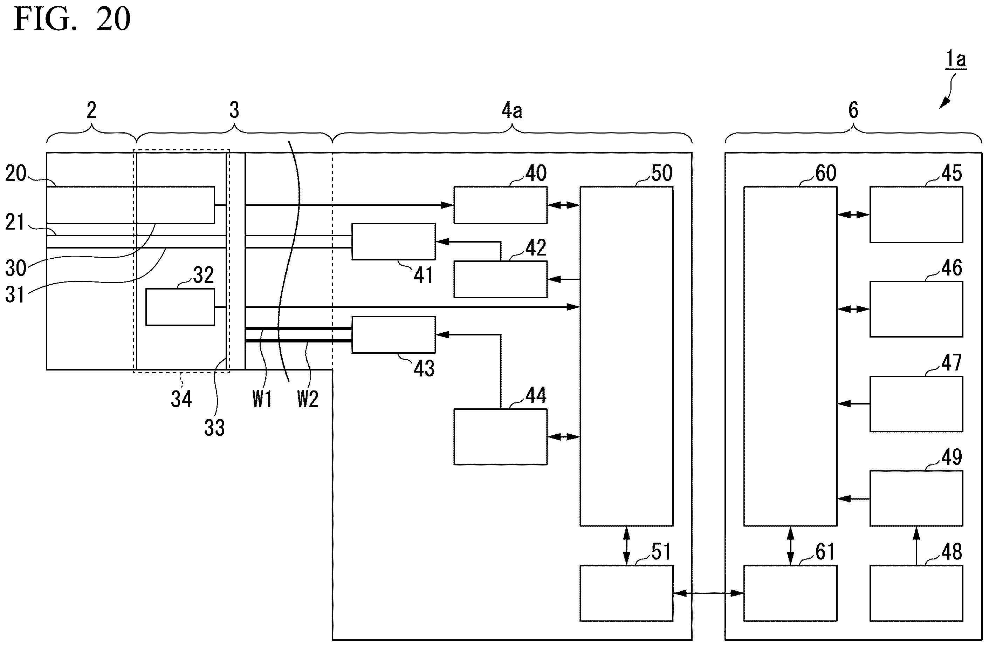

A first modified example of the first embodiment of the present invention will be described. FIG. 20 shows an example of the configuration of an endoscope system 1a according to the first modified example of the first embodiment. The same parts as those shown in FIG. 1 will not be described. The same blocks as those shown in FIG. 1 are referred to by the same reference signs as shown in FIG. 1.

The endoscope system 1a shown in FIG. 20 includes an optical adapter 2, an insertion unit 3, a main body 4a, and a base unit 6.

The optical adapter 2 shown in FIG. 20 is the same as the optical adapter 2 shown in FIG. 1. The insertion unit 3 shown in FIG. 20 is the same as the insertion unit 3 shown in FIG. 1.

The main body 4 shown in FIG. 1 is changed to the main body 4a shown in FIG. 20. The main body 4a includes an image processing unit 40, a light source 41, a light source control unit 42, a motor unit 43, a bending control unit 44, a control unit 50, and a communication unit 51. The communication unit 51 includes a communication circuit and executes wired communication or wireless communication for bending control or the like with the base unit 6.

The base unit 6 includes a display 45, a memory unit 46, an operation unit 47, a bending joystick 48, an A/D converter 49, a control unit 60, and a communication unit 61. The control unit 60 controls each unit of the base unit 6. The communication unit 61 includes a communication circuit and executes wired communication or wireless communication for bending control or the like with the main body 4a.

At least one of the control unit 50 and the control unit 60 executes the bending control process shown in FIG. 4. When information needs to be shared by the main body 4a and the base unit 6, the control unit 50 and the control unit 60 execute communication via the communication unit 51 and the communication unit 61.

The endoscope system according to each aspect of the present invention may include two or more control units. Some or all of the two or more control units may execute the bending control process in cooperation. Accordingly, the functions of the control unit 50 shown in FIG. 1 may be distributed to the two or more control units.

The two or more control units may sequentially execute processing. For example, a first control unit may execute part of the bending control process and output a process result to a second control unit. The second control unit may execute the other of the bending control process based on the process result. Alternatively, the two or more control units may execute processing at the same time.

The endoscope system according to each aspect of the present invention may include an external device. For example, the external device is a personal computer, a tablet terminal, or a cloud server over a network. The control unit 50 and one or more control units of the external device may cooperatively execute the bending control process. Alternatively, one or more control units of the external device may execute the entire bending control process.

In the first modified example of the first embodiment, similarly to the first embodiment, the endoscope system 1a does not require a user's complicated operations and reduce foreign matter in contact with the observation optical system.

### (Second modified example of first embodiment)

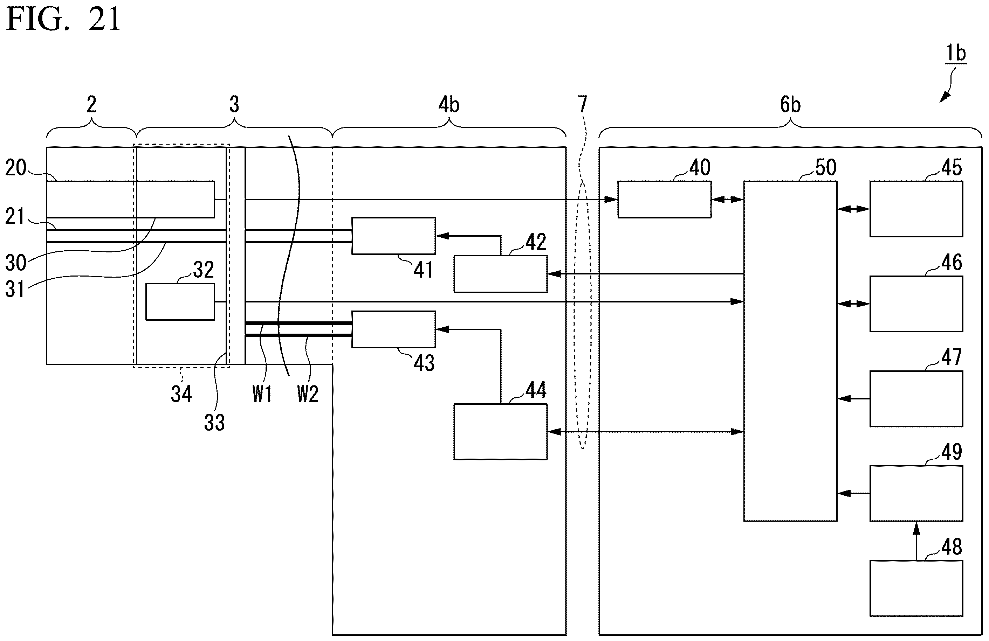

A second modified example of the first embodiment of the present invention will be described. FIG. 21 shows an example of the configuration of an endoscope system 1b according to the second modified example of the first embodiment. The same parts as those shown in FIG. 1 or 20 will not be described. The same blocks as those shown in FIG. 1 or 20 are referred to by the same reference signs as shown in FIG. 1 or 20.

The endoscope system 1b shown in FIG. 21 includes an optical adapter 2, an insertion unit 3, a main body 4b, and a base unit 6b. The main body 4b and the base unit 6b are connected via a cable 7.

The optical adapter 2 shown in FIG. 21 is the same as the optical adapter 2 shown in FIG. 1. The insertion unit 3 shown in FIG. 21 is the same as the insertion unit 3 shown in FIG. 1.

The main body 4 shown in FIG. 1 is changed to the main body 4b shown in FIG. 21. The main body 4b includes a light source 41, a light source control unit 42, a motor unit 43, and a bending control unit 44. The base unit 6b includes an image processing unit 40, a display 45, a memory unit 46, an operation unit 47, a bending joystick 48, an A/D converter 49, and a control unit 50.

In the second modified example of the first embodiment, similarly to the first embodiment, the endoscope system 1b does not require a user's complicated operations and reduce foreign matter in contact with the observation optical system.

### (Second embodiment)

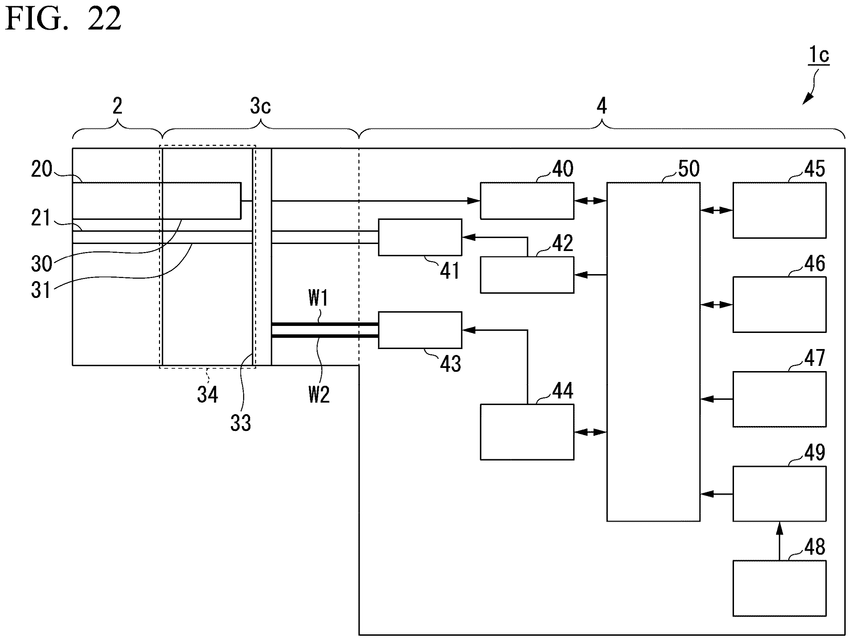

A second embodiment of the present invention will be described. FIG. 22 shows the configuration of an endoscope system 1c according to the second embodiment. The same parts as those shown in FIG. 1 will not be described. The same blocks as those shown in FIG. 1 are referred to by the same reference signs as shown in FIG. 1.

The endoscope system 1c shown in FIG. 22 includes an optical adapter 2, an insertion unit 3c, and a main body 4. The optical adapter 2 shown in FIG. 22 is the same as the optical adapter 2 shown in FIG. 1. The main body 4 shown in FIG. 22 is the same as the main body 4 shown in FIG. 1.

The insertion unit 3 shown in FIG. 1 is changed to the insertion unit 3c. The insertion unit 3c includes an imaging device 30, a light guide 31, and a bending portion 33. The insertion unit 3c does not include the sensor 32 shown in FIG. 1.

The control unit 50 determines the gravitational direction in the distal end portion 34 using an image generated by the imaging device 30. The control unit 50 outputs a control signal for bending the insertion unit 3c in the direction opposite to the gravitational direction to the bending control unit 44.

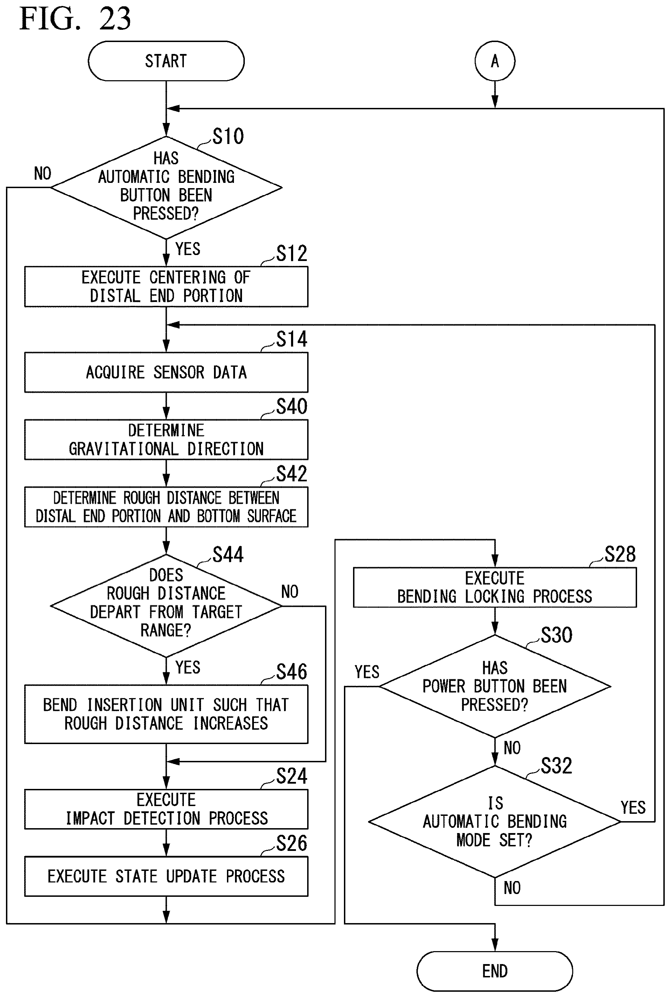

FIG. 23 shows an example of a procedure of a bending control process executed by the endoscope system 1c. The operation of the endoscope system 1c in the bending control process will be described with reference to FIG. 23. The same processes those as shown in FIG. 4 will not be described.

After Step S14, the control unit 50 determines the gravitational direction using an image generated by the imaging device 30 (Step S40).

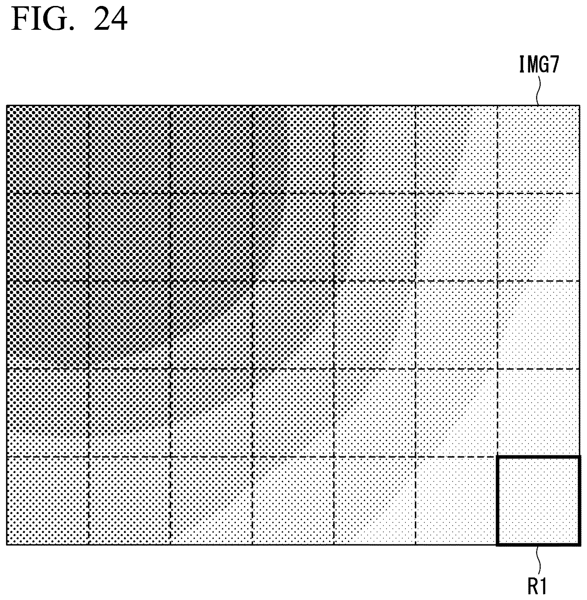

Details of Step S40 will be described. The control unit 50 determines a brightness of the image generated by the imaging device 30. FIG. 24 shows an example of the image generated by the imaging device 30. For example, the control unit 50 divides an image IMG7 shown in FIG. 24 into two or more regions and calculates a luminance value of each region based on pixel values of the image IMG7. The image IMG7 is divided into 35 regions having the same size.

The control unit 50 identifies a region having the greatest luminance value. Light reflected by the inside of the pipe is incident on the imaging device 30. Light reflected by the bottom surface of the pipe is the brightest. The brightest region in the image IMG7 corresponds to the bottom surface. For example, a region R1 shown in FIG. 24 has the highest luminance. The control unit 50 determines the position of the region R1 in the image IMG7. The region R1 is located on the right-down side of the center of the image IMG7. The control unit 50 determines that the gravitational direction is close to the right-down side in the image IMG7.

After Step S40, the control unit 50 determines a rough distance between the distal end portion 34 and the bottom surface of the pipe. The rough distance corresponds to the angle between the forward direction of the distal end portion 34 and the gravitational direction (Step S42).

Details of Step S42 will be described. When the distal end portion 34 is close to the bottom surface of the pipe, a bright region in the image generated by the imaging device 30 is wide. When the distal end portion 34 is far from the bottom surface of the pipe, the bright region in the image is narrow. That is, the area of the bright region in the image corresponds to the rough distance between the distal end portion 34 and the bottom surface of the pipe. For example, the control unit 50 compares the luminance value of each region in the image IMG7 shown in FIG. 24 with a reference value stored in the memory unit 46 and identifies a region having a luminance value greater than the reference value. The control unit 50 determines the number of identified regions. The number of regions indicates the area of the bright regions in the image IMG7 and corresponds to the rough distance between the distal end portion 34 and the bottom surface of the pipe.

The memory unit 46 stores a target range of the rough distance between the distal end portion 34 and the bottom surface of the pipe in advance. For example, the target range is greater than or equal to a first number. Alternatively, the target range is greater than or equal to the first number and less than or equal to a second number greater than the first number. After Step S42, the control unit 50 acquires the target range from the memory unit 46. The control unit 50 determines whether the rough distance identified in Step S42 departs from the target range (Step S44).

When the control unit 50 determines that the rough distance does not depart from the target range, that is, the rough distance is included in the target range in Step S44, Step S24 is executed. When the control unit 50 determines that the rough distance departs from the target range, that is, the rough distance is not included in the target range in Step S44, the control unit 50 outputs a control signal for bending the insertion unit 3c in the direction opposite to the gravitational direction such that the rough distance increases to the bending control unit 44. For example, when the gravitational direction is the right-down side in the image generated by the imaging device 30, a direction in which the insertion unit 3c is bent is the right-down side. The bending control unit 44 controls the motor unit 43 in accordance with the control signal and bends the insertion unit 3c in the direction opposite to the gravitational direction such that the rough distance increases. When the rough distance increases, the angle between the forward direction of the distal end portion 34 and the gravitational direction increases (Step S46). After Step S46, Step S24 is executed.

A stereo optical adapter having two fields of view may be used as the optical adapter 2. The stereo optical adapter includes a first optical system and a second optical system corresponding to two fields of view. The first optical system and the second optical system form two optical images of a subject on the imaging device 30. The imaging device 30 generates a stereo image corresponding to a first optical image and a second optical image. The stereo image includes a pair of two images (a first image and a second image). That is, the stereo image includes an image of the subject seen from a first viewpoint and an image of the subject seen from a second viewpoint.

In Step S42, the control unit 50 may calculate the distance between the distal end portion 34 and the bottom surface of the pipe by executing stereo measurement using two images included in the stereo image. For example, the control unit 50 may identify a first pixel in the brightest region in one of the two images and identify a second pixel corresponding to the first pixel in the other of the two images. The control unit 50 may calculate a three-dimensional distance from the distal end portion 34 to a point in a space corresponding to the first pixel based on coordinates of the first pixel and the second pixel. The three-dimensional distance indicates the distance between the distal end portion 34 and the bottom surface of the pipe.

Halation may occur in the image generated by the imaging device 30. In stereo measurement using a pixel in which halation occurs, a measurement result is likely to include an error. The control unit 50 may identify regions in which halation does not occur in the image generated by the imaging device 30 and calculate the distance between the distal end portion 34 and the bottom surface of the pipe based on a pixel in the brightest region out of the identified regions.

Similarly to the first embodiment, the control unit 50 may generate information indicating the relationship between the gravitational direction and the reference direction in the imaging surface of the imaging device 30 in Step S40. The control unit 50 may superimpose the information on the image generated by the imaging device 30 and output the image on which the information has been superimposed to the display 45.

Each aspect of the present invention may include the following modified example. The endoscope system 1c includes the imaging device 30 (an image sensor) that generates an image. The control unit 50 acquires an image output from the imaging device 30 and determines the gravitational direction in the distal end portion 34 based on the image.

Each aspect of the present invention may include the following modified example. The control unit 50 determines the gravitational direction based on a brightness at two or more positions in the image generated by the imaging device 30.

Each aspect of the present invention may include the following modified example. The control unit 50 determines the distance between the distal end portion 34 and a subject into which the insertion unit 3c is inserted using the image generated by the imaging device 30. The control unit 50 outputs the control signal for bending the insertion unit 3c such that the distance increases.

The endoscope system 1c according to the second embodiment analyzes the image generated by the imaging device 30 in the automatic bending mode and bends the insertion unit 3c such that the angle between the forward direction of the distal end portion 34 and the gravitational direction increases. Accordingly, the endoscope system 1c does not require a user's complicated operations and reduce foreign matter in contact with the observation optical system. A user can efficiently perform inspection.

While exemplary embodiments of the present invention have been described above, the present invention is not limited to these embodiments and modified examples thereof. Addition, omission, substitution, and other modifications of constituents are possible without departing from the gist of the present invention. The present invention is not limited to the aforementioned description and is defined by only the appended claims.

### REFERENCE SIGNS LIST

1, 1a, 1b, 1c Endoscope system
2 Optical adapter
3, 3c Insertion unit
4, 4a, 4b Main body
6, 6b Base unit
7 Cable
20 Imaging lens unit
21 Illumination lens unit
30 Imaging device
31 Light guide
32 Sensor
33 Bending portion
34 Distal end portion
40 Image processing unit
41 Light source
42 Light source control unit
43 Motor unit
44 Bending control unit
45 Display
46 Memory unit
47 Operation unit
48 Bending joystick
49 A/D converter
50, 60 Control unit
51, 61 Communication unit

## Claims

1. An endoscope system comprising:
an insertion unit that includes a sensor at a distal end and that is bendable; and
a control unit configured to:
receive a mode signal indicating a mode in which the insertion unit is automatically bent;
acquire data output from the sensor;
determine a gravitational direction at the distal end based on the data; and
output a control signal for bending the insertion unit such that an angle between a forward direction of the distal end and the gravitational direction increases after the mode signal has been received.

2. The endoscope system according to claim 1, wherein the control unit is configured to output a state control signal for controlling a bending state of the insertion unit such that the angle is fixed after the control signal has been output.

3. The endoscope system according to claim 1, wherein the control unit is configured to output the control signal for bending the insertion unit in a first direction and a second direction perpendicular to the first direction such that the angle increases according to an amount of twist of the insertion unit.

4. The endoscope system according to claim 1, wherein the control unit is configured to generate the control signal based on a predetermined value stored in a memory, and
wherein the predetermined value indicates a target value of the angle or a target value of an amount of increase of the angle.

5. The endoscope system according to claim 4, wherein the memory is configured to store two or more values as the predetermined value, and
wherein the control unit is configured to acquire any one of the two or more values from the memory.

6. The endoscope system according to claim 5, wherein the control unit is configured to acquire the predetermined value corresponding to a type of an optical adapter attached to the distal end from the memory.

7. The endoscope system according to claim 4, wherein the predetermined value is changeable.

8. The endoscope system according to claim 1, wherein the sensor is an acceleration sensor, and
wherein the data indicates acceleration of the distal end.

9. The endoscope system according to claim 8, further comprising an image sensor configured to generate an image,
wherein the control unit is configured to:
determine a posture of the distal end based on the gravitational direction;
superimpose information indicating the posture on the image; and
output the image on which the information has been superimposed to a display.

10. The endoscope system according to claim 1, wherein the sensor is an image sensor configured to generate an image as the data.

11. The endoscope system according to claim 10, wherein the control unit is configured to determine the gravitational direction based on a brightness at two or more positions in the image.

12. The endoscope system according to claim 11, wherein the control unit is configured to:
determine a distance between the distal end and a subject into which the insertion unit is inserted using the image; and
output the control signal for bending the insertion unit such that the distance increases.

13. The endoscope system according to claim 1, wherein the control unit is configured to:
output an image for displaying a graphical user interface associated with setting of the mode to a display; and
receive the mode signal when it is instructed to set the mode via the graphical user interface.

14. The endoscope system according to claim 1, further comprising an image sensor configured to generate an image,
wherein the control unit is configured to:
calculate a component in the gravitational direction on an imaging surface of the image sensor;
calculate an angle between a direction of the component on the imaging surface and a reference direction on the imaging surface; and
correct the image by rotating the image by the angle.

15. A control method comprising:
receiving a mode signal indicating a mode in which an insertion unit including a sensor at a distal end and being bendable is automatically bent;
acquiring data output from the sensor;
determining a gravitational direction at the distal end based on the data; and
outputting a control signal for bending the insertion unit such that an angle between a forward direction of the distal end and the gravitational direction increases after the mode signal has been received.

16. A program causing a computer to execute:
a step of receiving a mode signal indicating a mode in which an insertion unit including a sensor at a distal end and being bendable is automatically bent;
a step of acquiring data output from the sensor;
a step of determining a gravitational direction at the distal end based on the data; and
a step of outputting a control signal for bending the insertion unit such that an angle between a forward direction of the distal end and the gravitational direction increases after the mode signal has been received.
